# EUROPEAN PATENT SPECIFICATION

(11) **EP 2 581 029 B1**
(45) Date of publication and mention of the grant of the patent: **31.12.2014**
(21) Application number: 11857389.8
(22) Date of filing: 08.11.2011
(51) Int. Cl.: A61B 1/00, A61B 1/04, A61B 6/03, A61B 1/267, A61B 8/08

(54) **MEDICAL DEVICE**
MEDIZINISCHE VORRICHTUNG
DISPOSITIF MÉDICAL

(30) Priority: 24.01.2011 JP 2011012103
(43) Date of publication of application: 17.04.2013
(73) Proprietor: OLYMPUS MEDICAL SYSTEMS CORP., Tokyo 151-0072 (JP)
(72) Inventor: ONISHI, Junichi, Tokyo 151-0072 (JP); AKIMOTO, Syunya, Tokyo 151-0072 (JP); ITO, Mitsuhiro, Tokyo 151-0072 (JP)
(74) Representative: Gunzelmann, Rainer
(86) International application number: PCT/JP2011/075686
(87) International publication number: WO 2012/101888

(56) References cited:
- WO-A1-2007/129493
- JP-A- 2004 089 483
- JP-A- 2005 304 937
- JP-A- 2006 181 110
- JP-A- 2010 517 632
- JP-B1- 4 728 456
- US-A1- 2008 207 997
- US-B1- 6 346 940

## Description

### Technical Field

Embodiments of the present invention relate to a medical device configured to be inserted into a lumen of a subject, and more particularly to a medical device that performs highly accurate inspection/treatment based on three-dimensional image data of a subject.

### Background Art

In recent years, diagnosis using a three-dimensional image has been widely performed. For example, three-dimensional image data inside a subject is acquired by picking up a tomographic image of the subject using an X-ray CT (Computed Tomography) apparatus, and diagnosis of a target site has been performed using the three-dimensional image data.

A CT apparatus continuously performs scanning on a subject in a helical mode (helical scan) by continuously moving the subject while continuously rotating an X-ray irradiation position and a detection position. Then, a three-dimensional image is created from a multiple of continuous two-dimensional tomographic images of the subject.

As one of three-dimensional images used for diagnosis, a three-dimensional image of the bronchus of the lung is known. A three-dimensional image of the bronchus is used for three-dimensionally figuring out a position of an abnormal portion at which a lung cancer and the like are suspected to exist, for example. Then, in order to check the abnormal portion by biopsy, a bronchoscope is inserted in the bronchus and a biopsy needle, biopsy forceps, or the like is protruded from a distal end portion of an insertion portion to take a sample of a tissue.

In tracts in a body which have multilevel bifurcations such as the bronchus, if an abnormal portion exists at a periphery of the bronchus, it is difficult to make the distal end portion reach in the vicinity of a target site precisely in a short time. Therefore, Japanese Patent Application Laid-Open Publication Nos. 2004-180940 and 2005-131042, for example, disclose an insertion navigation system which creates a three-dimensional image of a tract in the subject based on three-dimensional image data of the subject, calculates a route to a target point along the tract on the three-dimensional image, and creates a virtual endoscopic image of the tract along the route based on the image data, to display the created virtual endoscopic image.

In addition, Japanese Patent Application Laid-Open Publication No. 2003-265408 discloses an endoscope guiding apparatus which displays a position of the endoscope distal end portion in a superimposed manner on a tomographic image.

Document US 6,346,940 B1 describes an image processing system displaying an endoscopic image of a three-dimensional object from a desired viewpoint position and viewpoint direction. The actual insertion of an endoscope tip into the subject can be carried out while viewing virtual images on a display unit of an image processing system and real images on a monitor of the electronic endoscope system. The image processing system comprises a guiding marker preparation unit, a shortest path computation unit, an operation path history control unit and an endoscopic image preparation unit.

However, in the known insertion navigation system, there has been a case in which it is not easy for an operator to confirm a whole image of an insertion route in an insertion operation. Also, in the case of displaying the position of the endoscope distal end portion in the superimposed manner on the tomographic image, it is necessary to use more than two tomographic images in order to confirm a three-dimensional position and it can not be said that good visibility is provided.

Therefore, in the known insertion navigation system, there has been a case where the distal end portion of the insertion portion can not be easily inserted to the target site.

An object of embodiments of the present invention is to provide a medical device having a distal end portion of an insertion portion capable of being inserted to a target site.

### Disclosure of Invention

### Means for Solving the Problem

The present invention is a medical device as defined by claim 1.

A medical device according to one embodiment of the present invention includes: insertion means to be inserted into a lumen of a subject; storing means configured to store previously acquired three-dimensional image data of the subject; position calculation means configured to calculate a position and a direction of a distal end portion of the insertion means in the lumen; route generation means configured to generate an insertion route for inserting the distal end portion to a target position through the lumen, based on the three-dimensional image; tomographic image generation means configured to generate a tomographic image based on the position and the direction of the distal end portion, from the three-dimensional image data; superimposed image generation means configured to generate a superimposed image in which the insertion route is superimposed on the tomographic image; and display means configured to display the superimposed image.

### Brief Description of the Drawings

FIG 1 a three-dimensional model view for illustrating a state where a medical device according to a first embodiment is inserted into the bronchus.
FIG 2 is a configuration diagram for illustrating a configuration of the medical device according to the first embodiment.
FIG 3A is an illustration diagram for illustrating a tomographic image.
FIG 3B is an illustration diagram for illustrating a tomographic image.
FIG 3C is an illustration diagram for illustrating a tomographic image.
FIG 3D is an illustration diagram for illustrating a tomographic image.
FIG 3E is an illustration diagram for illustrating a tomographic image.
FIG 3F is an illustration diagram for illustrating a tomographic image.
FIG 4 is a flowchart for illustrating a flow of processing steps performed by the medical device according to the first embodiment.
FIG 5 is an illustration diagram for illustrating a target position setting screen of the medical device according to the first embodiment.
FIG 6 is an illustration diagram for illustrating the target position setting screen of the medical device according to the first embodiment.
FIG 7 is an illustration diagram for illustrating a navigation screen of the medical device according to the first embodiment.
FIG 8 is a three-dimensional model view for illustrating a superimposed image displayed by the medical device according to the first embodiment.
FIG 9 shows an example of a superimposed image displayed by the medical device according to the first embodiment.
FIG 10 shows an example of the superimposed image displayed by the medical device according to the first embodiment.
FIG. 11 shows an example of the superimposed image displayed by the medical device according to the first embodiment.
FIG 12 shows an example of the superimposed image displayed by the medical device according to the first embodiment.
FIG 13 shows an example of the superimposed image displayed by the medical device according to the first embodiment.
FIG 14 is an illustration diagram for illustrating a navigation screen of a medical device according to a second embodiment.
FIG 15 is a configuration diagram for illustrating a configuration of a medical device according to a modified example 1 of the second embodiment.
FIG 16A is a configuration diagram for illustrating a superimposed image displayed by the medical device according to the modified example 1 of the second embodiment.
FIG 16B is a configuration diagram for illustrating the superimposed image displayed by the medical device according to the modified example 1 of the second embodiment.
FIG 17 is a configuration diagram for illustrating a configuration of a medical device according to a modified example 2 of the second embodiment.
FIG 18A is an illustration diagram for illustrating a superimposed image displayed by the medical device according to the modified example 2 of the second embodiment.
FIG 18B is an illustration diagram for illustrating the superimposed image displayed by the medical device according to the modified example 2 of the second embodiment.
FIG 19A is an illustration diagram for illustrating an auxiliary route displayed by the medical device according to the modified example 2 of the second embodiment.
FIG 19B is an illustration diagram for illustrating the auxiliary route displayed by the medical device according to the modified example 2 of the second embodiment.
FIG 20 is a configuration diagram for illustrating a configuration of a medical device according to a third embodiment.
FIG 21 is a configuration diagram for illustrating a configuration of a medical device according to a modified example of the third embodiment.
FIG 22 is an illustration diagram for illustrating a distal end portion of an insertion portion of a medical device according to a fourth embodiment.
FIG 23 shows an example of a superimposed image displayed by the medical device according to the fourth embodiment.
FIG 24 is a flowchart for illustrating a flow of processing steps performed by the medical device according to the fourth embodiment.
FIG 25 is an illustration diagram for illustrating a distal end portion of an insertion portion of a medical device according to a fifth embodiment.
FIG 26 shows an example of a superimposed image displayed by the medical device according to the fifth embodiment.

### Best Mode for Carrying Out the Invention

### <First Embodiment>

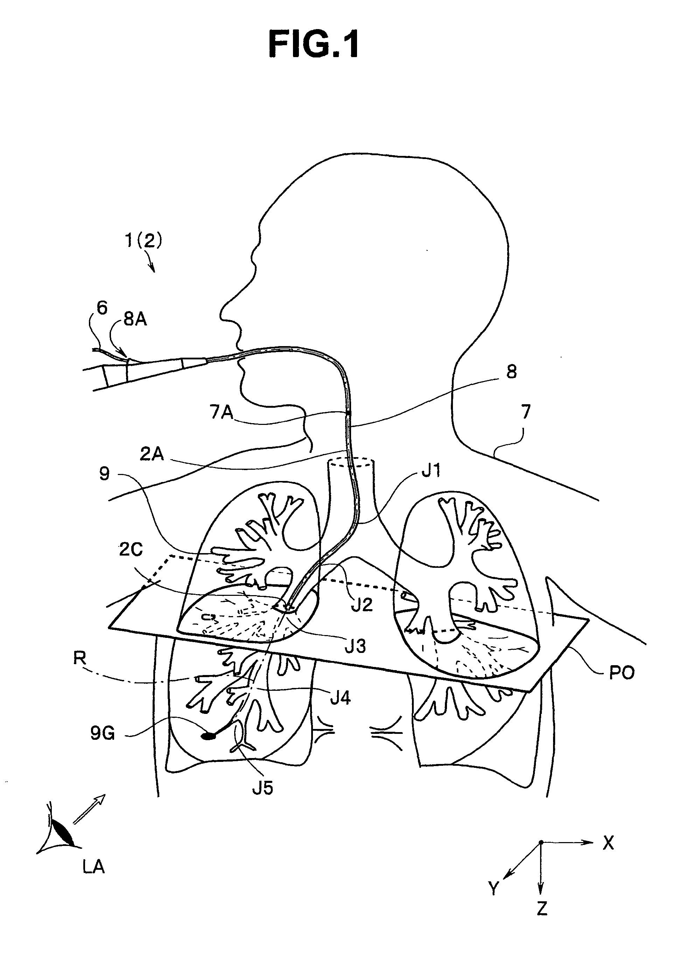

Hereinafter, a medical device 1 according to a first embodiment of the present invention will be described with reference to drawings. As shown in FIG 1, the medical device 1 navigates a distal end portion 2C of an insertion portion 2A of an endoscope apparatus 2 so as to insert the distal end portion from a pharynx portion 7A of a subject 7, which is an insertion start position, through a bronchus 9 having a plurality of bifurcation portions J1 to J5, to a target site 9G as a target position. FIG 1 is a three-dimensional model view showing a state where the insertion portion 2A is inserted toward the target site 9G The insertion portion 2A includes a channel 8 which is inserted through the inside of the insertion portion, and a treatment instrument 6 inserted from a channel insertion port 8A is protruded from the distal end portion 2C to perform biopsy on the target site 9G Note that, in the drawings to be described below, a Z-axis direction is a body axis of the subject 7, an X-axis direction is a left/light direction of the subject 7, and a Y-axis direction is a front/rear direction of the subject 7.

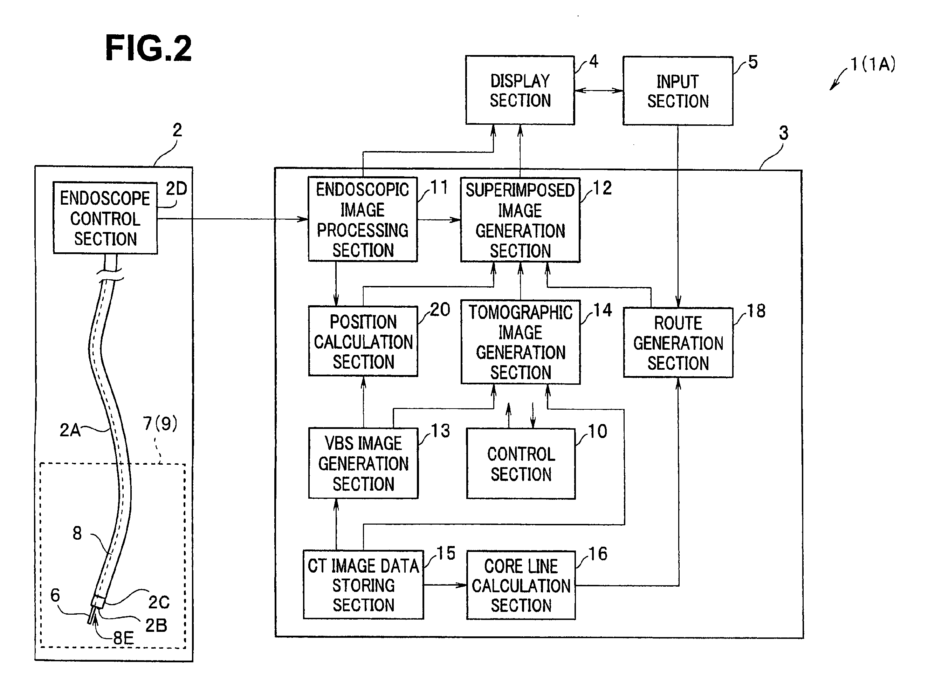

As described later, in the medical device 1, during an insertion operation, a superimposed image PW1 as a three-dimensional model image showing a three-dimensional space on which a tomographic image (oblique image) PO of a plane which includes a position of the distal end portion 2C at that time and which is perpendicular to the direction of the distal end portion 2C and a three-dimensional insertion route R are displayed in a superimposed manner is displayed on a display section 4 (See FIG 2). A line of sight LA (viewpoint position, direction of line of sight, and roll angle of line of sight) for the three-dimensional model image can be arbitrarily set by an operator.

As the position of the distal end portion 2C changes, that is, the insertion operation advances, the tomographic image PO to be displayed is automatically updated. Note that a position display mark P2C which shows the position of the distal end portion 2C of the insertion portion 2A is displayed on the tomographic image PO in a superimposed manner.

Next, a configuration of the medical device 1 will be described with reference to FIG 2. The medical device 1 includes the endoscope apparatus 2, a main body section 3 for performing insertion support, the display section 4 as display means, and an input section 5 as input means.

The endoscope apparatus 2 is a bronchoscope including the insertion portion 2A which is insertion means having an image pickup section 2B as image pickup means disposed at the distal end portion 2C, and an endoscope control section 2D which controls the insertion portion 2A and the like. The insertion portion 2A includes inside thereof the channel 8 through which the treatment instrument 6 is insertable. When the distal end portion 2C is inserted close to the target site 9G, the treatment instrument 6 is protruded from a channel opening 8E of the distal end portion 2C and biopsy is performed.

The main body section 3 includes: an endoscopic image processing section 11; a superimposed image generation section 12 as superimposed image generation means; a position calculation section 20 as position calculation means; a virtual endoscopic image (Virtual Bronchus Scope image: hereinafter also referred to as "VBS image") generation section 13; a tomographic image generation section 14 as tomographic image generation means; a CT image data storing section 15 as storing means; a core line calculation section 16 as core line calculation means; a route generation section 18 as route generation means; and a control section 10 as control means.

The control section 10 controls the whole navigation. The endoscopic image processing section 11 processes an image picked up by the image pickup section 2B and outputs endoscopic image (hereinafter, also referred to as "real image"). The CT image data storing section 15 stores three-dimensional image data of the subject 7 which was previously acquired by using a CT apparatus. The VBS image generation section 13 generates, based on the three-dimensional image data, a VBS image which uses the position, the direction, and the roll angle (hereinafter, also referred to as "position and the like") of the distal end portion 2C as line-of-sight parameters.

The position calculation section 20 calculates the position and the like of the distal end portion 2C of the insertion portion 2A inserted into the bronchus 9. The core line calculation section 16 calculates a core line S of the bronchus 9 based on the three-dimensional image data. Here, the core line S shows information on a line connecting gravity center points on a vertical plane in the tract direction of the bronchus 9, that is, the longitudinal direction of the lumen. As the core line S, information on the center line which connects the center points on the vertical plane in the tract direction of the lumen, and the like may be used.

The route generation section 18 generates, based on the three-dimensional image data, the insertion route R along the core line S to the target site 9G which is a target position set by the operator using the input section 5.

The tomographic image generation section 14 generates, based on the three-dimensional image data, the tomographic image PO of a plane which includes the three-dimensional position of the distal end portion 2C calculated by the position calculation section 20 and which is perpendicular to the direction of the distal end portion 2C.

The superimposed image generation section 12 generates a three-dimensional space on which the three-dimensional insertion route R is superimposed on the tomographic image PO generated by the tomographic image generation section 14, as a superimposed image PW1 which is a three-dimensional model image observed along a predetermined line of sight LA.

The display section 4 displays a navigation image including at least one of the real image and the VBS image, and the superimposed image PW1, during insertion operation.

Note that each of the above-described constituent elements of the main body section 3 is not necessarily a separate hardware but may be a program to be read by the CPU to operate, for example.

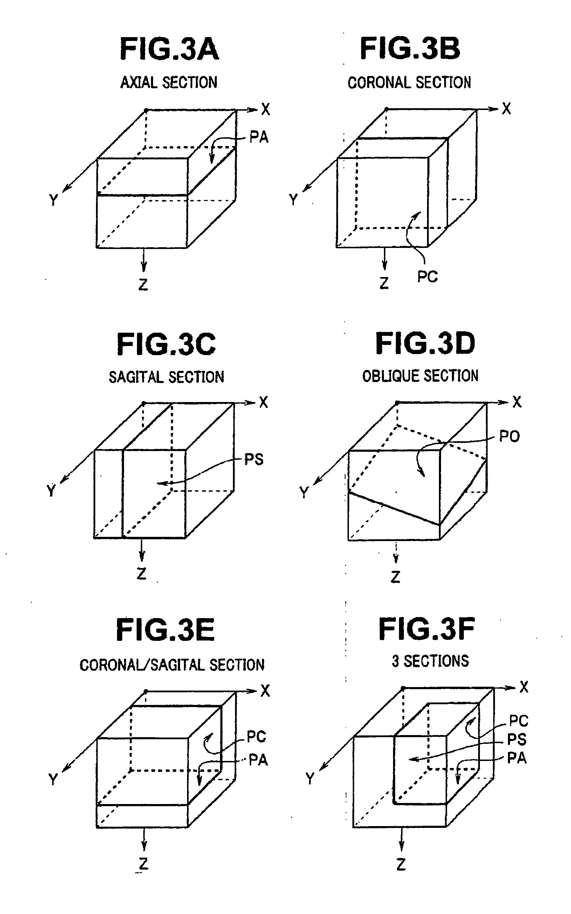

Hereinafter, description will be made on the tomographic image generated by the tomographic image generation section 14 with reference to FIGS. 3A to 3F.

An axial image PA shown in FIG 3A is an image of an XY plane perpendicular to the body axis of the subject 7, a coronal image PC shown in FIG 3B is an image of an XZ plane facing the subject 7, and a sagital image PS is an image of a YZ plane in a side surface direction of the subject 7. In addition, an oblique image PO shown in FIG 3D is an image of an arbitrary plane.

Furthermore, the composite tomographic image shown in FIG 3E includes two planes PA and PC perpendicular to each other. The composite tomographic image including two planes perpendicular to each other may be composed of a combination of images of other planes. In addition, the composite tomographic image may be composed of the oblique image PO and an image of the plane perpendicular to the oblique image. The composite tomographic image shown in FIG 3F is an example of a composite tomographic image including three planes perpendicular to one another.

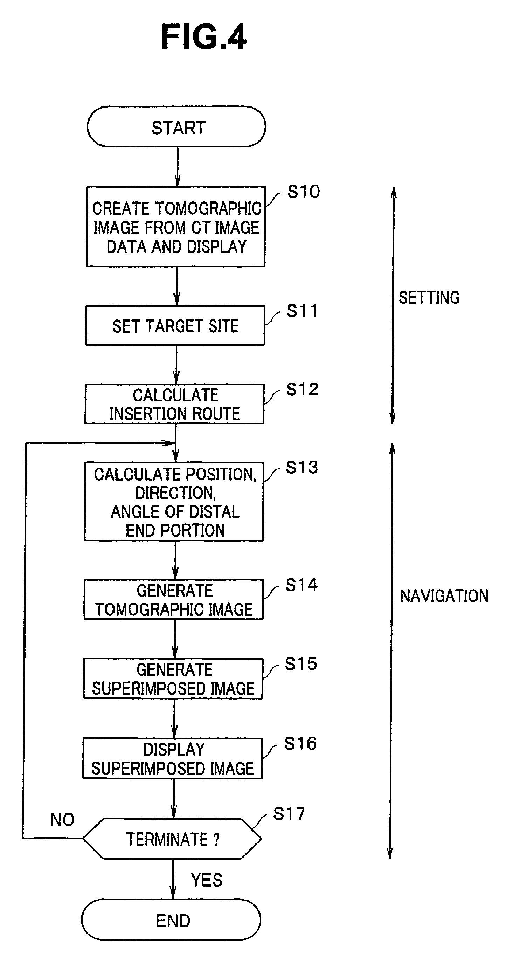

Next, description will be made on a flow of processing steps performed by the medical device 1 with reference to the flowchart in FIG 4.

### «Setting Operation Mode»

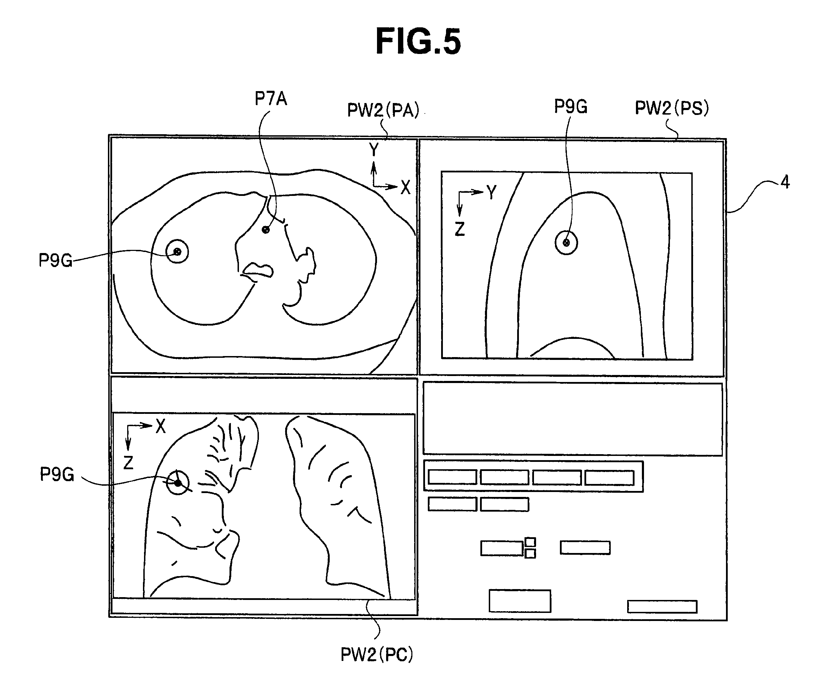

First, a target position setting screen shown in FIG 5 is displayed on the display section 4. The target position setting screen displays the axial image PA, the coronal image PC, and the sagital image PS.

### <Step S10> Tomographic Image Creation

Three-dimensional coordinates representing the target site 9G have to be set using the display section 4 which displays a two-dimensional image. Therefore, at first, three kinds of tomographic images, i.e., the axial image PA, the coronal image PC, and the sagital image PS are generated from the three-dimensional image data of the subject. The tomographic image for target position setting is created with the body axis set as the Z axis, for example.

### <Step S11> Target Position Setting

As shown in FIG 5, the target site 9G is set using the target position setting screen displayed on the display section 4. To this end, a target position mark P9G indicating the target position is superimposed on each of the axial image PA, the coronal image PC, and the sagital image PS, which are displayed on the target position setting screen. Note that, in the example shown in FIG 5, a start position mark P7A indicating the position of the pharynx portion 7A as the insertion start position is located inside the display range of the axial image PA but is located outside the display ranges of the coronal image PC and the sagital image PS.

When the operator moves the target position mark P9G displayed in a superimposed manner on any of the tomographic images, using a mouse or the like as input means, in accordance with the movement, the target position marks 9G displayed on other tomographic images also move.

Note that also the insertion start position may be settable by moving operation of the start position mark P7A. In addition, the target position does not have to be a point, but may be a target region having a predetermined volume. Furthermore, in order to set the target position more precisely, the tomographic images may be displayed in an enlarged manner.

### <Step S12> Insertion Route Calculation

When the target site 9G is set, the route generation section 18 generates the insertion route R from the pharynx portion 7A as the insertion start position to the target site 9G as the target position, based on the three-dimensional image data stored in the CT image data storing section 15. The insertion route R is a core line leading to the target site 9G, which is a part of the core line S connecting the gravity center points or the center points of the luminal cross sections in the three-dimensional image data.

The route generation section 18 may generate a plurality of insertion routes, and the selection of a route may be left to the operator. That is, when the target site 9G is located between a plurality of lumens, or the target site 9G is a site having a volume equal to or larger than a predetermined volume, for example, a plurality of insertion routes are calculated.

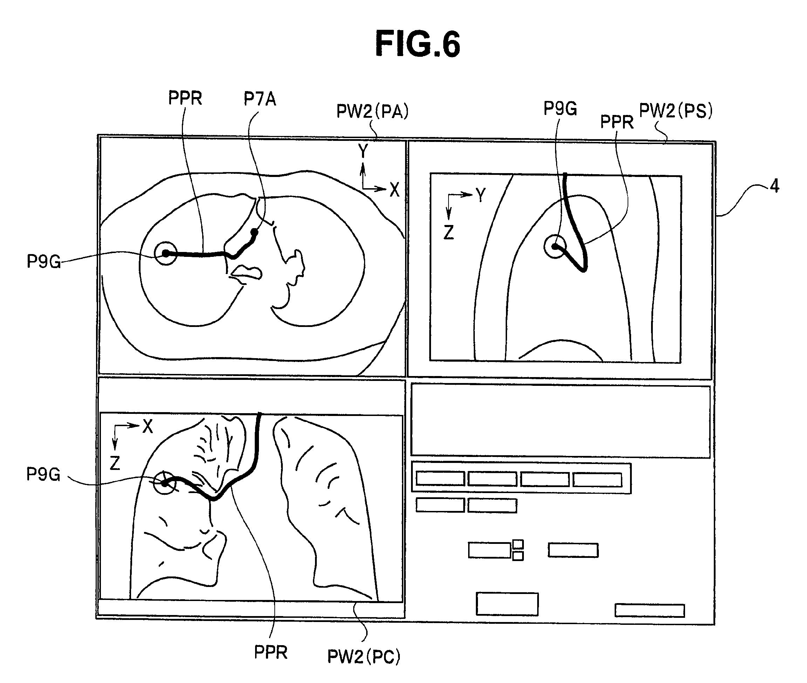

As shown in FIG 6, when the insertion route R is calculated, superimposed images PW2 in which route images PPR showing the insertion route R are displayed on the tomographic images are displayed. Here, the route images PPR are images acquired by reflecting the three-dimensional insertion route R on the planes of the respective tomographic images.

On the other hand, the VBS image generation section 13 generates VBS images of the bifurcation portions J1 to J4 on the insertion route R and thumbnail images as reduced images of the respective VBS images.

### «Insertion Navigation Mode»

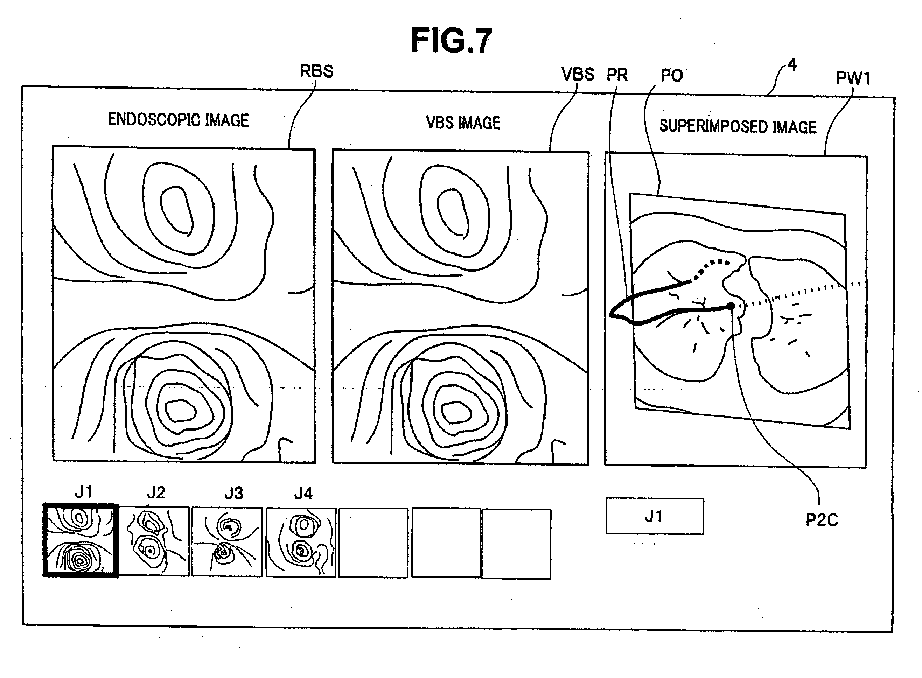

As shown in FIG 7, when the insertion operation is started, a navigation screen is displayed on the display section 4. On the navigation screen, the real image RBS, the VBS image VBS, the superimposed image PW1, the thumbnail images, and bifurcation portion numbers are displayed. Note that FIG. 7 is an example of the navigation screen at the time that the distal end portion 2C is located at the first bifurcation portion J1 among the four bifurcation portions. The thumbnail images display the reduced images of the four bifurcation portions J1 to J4, and the bifurcation portion number J1 is largely displayed.

### <Step S13> Calculation of Position, Direction, and Roll Angle of Distal End Portion

The position calculation section 20 calculates the position and the like of the distal end portion 2C on a real-time basis, or at a predetermined time interval.

Then, the position calculation section 20 controls the VBS image generation section 13 to generate a VBS image similar to the real image photographed by the CCD (2B). That is, the VBS image generation section 13 generates a VBS image which uses the position, the direction and the roll angle (X1, Y1, Z1, a1, e1, r1) as line-of-sight parameters. In this embodiment, (X, Y, Z) represent three-dimensional coordinate values, (a) represents an azimuth angle, (e) represents an elevation angle, and (r) represents a roll angle.

Then, the position calculation section 20 compares the VBS image and the real image to calculate a similarity therebetween. The calculation of the similarity between the images is performed by a publicly known processing, and may be performed by using either a matching at a pixel data level or a matching at a level of features extracted from the images.

The matching processing between the real image and the VBS image is performed in the unit of frames of the real image. Therefore, the actual comparison processing is performed with the similarity between a still endoscopic image and the VBS image as a reference.

When an error e between the images calculated based on the similarity calculated by comparing the real image with the VBS image B is larger than a predetermined admissible error e0, the position calculation section 20 outputs line-of-sight parameters whose values have been changed to the VBS image generation section 13. The VBS image generation section 13 generates the next one VBS image according to the new line-of-sight parameters.

By performing the above-described processing repeatedly, that is, by changing the line-of-sight parameters, the VBS image B generated by the VBS image generation section 13 gradually becomes an image similar to the real image, and after repeating the processing a several times, the error e between the images becomes equal to or smaller than the admissible error e0.

Then, the position calculation section 20 calculates the information on the position and the like (X, Y, Z, a, e, r) of the distal end portion 2C based on the line-of-sight parameter of the VBS image similar to the real image. That is, the position, the direction and the roll angle of the distal end portion 2C calculated by the position calculation section 20, are more precisely the line-of-sight position, the line-of-sight direction, and the roll angle of the image pickup section 2B disposed at the distal end portion 2C.

### <Step S14> Tomographic Image Generation

The tomographic image generation section 14 generates a tomographic image of the plane P including the three-dimensional position (X, Y, Z) of the distal end portion 2C calculated by the position calculation section 20. Note that the operator can select a desired image from the tomographic images shown in FIGS. 3A to 3F. A preferred tomographic image is the oblique image PO of the plane perpendicular to the direction of the distal end portion 2C shown in FIG 3D, or a composite tomographic image including the oblique image PO. This is because the operator can most easily recognize the position and the direction of the distal end portion 2C.

### <Step S15> Superimposed Image Generation

The superimposed image generation section 12 generates the superimposed image PW1 in which the insertion route R is superimposed on the tomographic image PO.

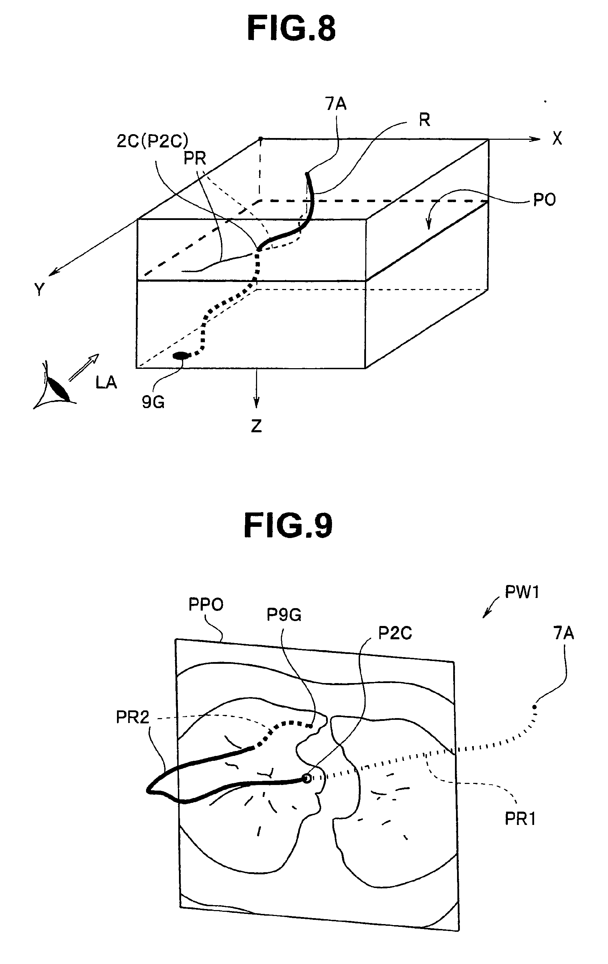

The three-dimensional model image, which is viewed along a desired line of sight LA, of the three-dimensional space on which the two-dimensional tomographic image PO and the three-dimensional insertion route R are arranged as shown in FIG 8 is the superimposed image PW1 shown in FIG 9.

The superimposed image PW1 shown in FIG 9 seems to be similar to each of the superimposed image PW2 on the target position setting screen shown in FIG 6. However, on each of the superimposed images PW2, the route image is two-dimensional route image acquired by projecting the three-dimensional insertion route R on each of the tomographic images, and each of the tomographic images is a tomographic image of the preset plane. That is, each of the superimposed images PW2 is a normal two-dimensional image.

In contrast, the superimposed image PW1 is a three-dimensional model image, and can be changed in a desired state by the operator arbitrarily changing the line of sight LA. For example, if the line of sight LA is set on the extension of the plane of the tomographic image PO, the tomographic image PO on the superimposed image PW1 is displayed with a line. In addition, in the superimposed image PW1, the tomographic image PO includes the distal end portion 2C. Therefore, the operator can acquire the information on the tissue around the distal end portion 2C.

Note that, in the superimposed image PW1, the point of the intersection between the route image PR showing the insertion route R and the tomographic image PO is the position of the distal end portion 2C at which the position display mark P2C is displayed.

In addition, the superimposed image generation section 12 shows the route image PR1 from the start position mark P7A showing the position of the pharynx portion 7A as the insertion start position to the position display mark P2C indicating the position of the distal end portion 2C with a distinguishable line which is different from the line representing the route image PR2 from the position display mark P2C to the target position mark P9G indicating the target position. That is, the route image PR1 is displayed with a dotted line, and the route image PR2 is mainly displayed with a solid line. Furthermore, the superimposed image generation section 12 displays a part of the route image PR2 with a dashed line, the part being located on the rear side of the tomographic image PO when viewed along the line of sight LA.

Note that the superimposed image generation section 12 may display the route image PR1 and the route image PR2 with different colors or different thicknesses in order to distinguish the route images from each other. In addition, the superimposed image generation section 12 does not have to display the route image PR1.

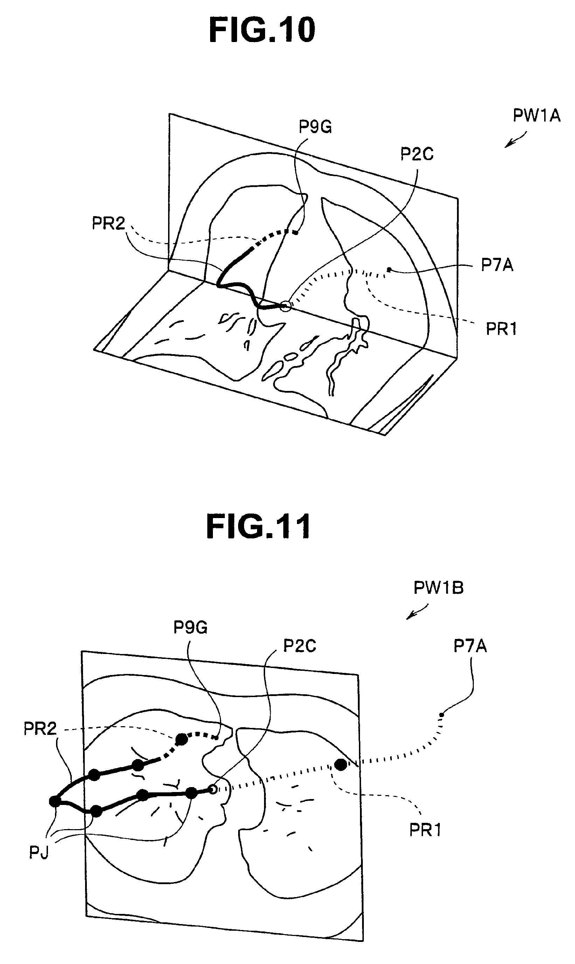

Furthermore, as shown in FIG 10, the superimposed image generation section 12 may generate a superimposed image PW 1A using a composite tomographic image as the tomographic image PO. The superimposed image PW1A is a three-dimensional model image including the composite tomographic image composed of the two planes PA and PC which are perpendicular to each other, as shown in FIG 3E, and the insertion route R. In the superimposed image PW1A, the distal end portion 2C is located on the cross line between the plane PA and the plane PC perpendicular to the direction of the distal end portion 2C.

Furthermore, as shown in FIG 11, the superimposed image generation section 12 may generate a superimposed image PW1B in which bifurcation portion display marks PJ1 to PJ4 which indicate the respective positions of the bifurcation portions are superimposed on the route image PR.

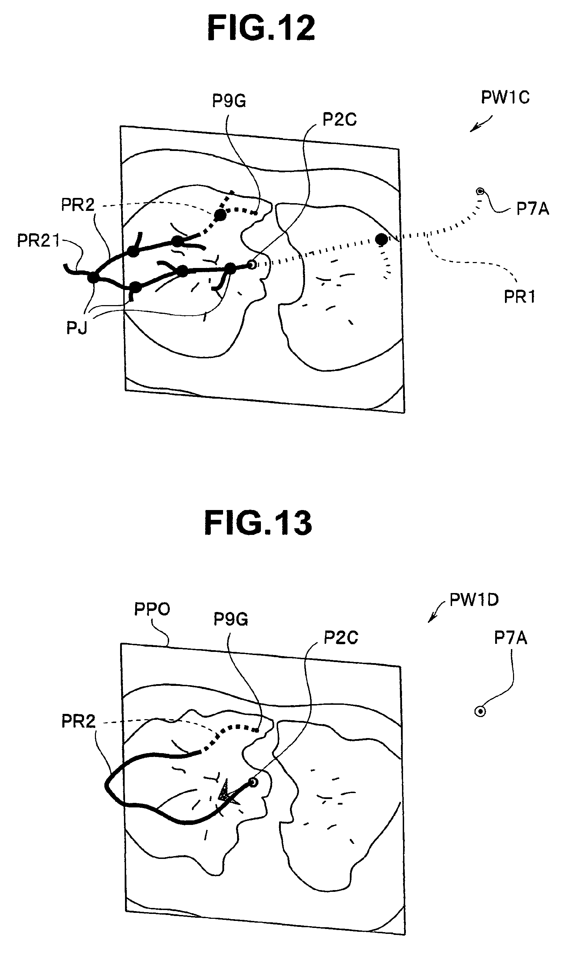

Furthermore, as shown in FIG 12, the superimposed image generation section 12 may generate a superimposed image PW1C in which the image PR2 of a part of the core line S other than the insertion route is superimposed as the route image PR. However, when many core lines S are displayed, it becomes difficult to recognize the insertion route. Therefore, it is preferable to perform display restriction for displaying only the core line S branched off once from the insertion route, as shown in FIG 12. As another display restricting method, only the core lines S of a predetermined number of bifurcations may be displayed, or the core lines S may be displayed by a predetermined length from the bifurcation portion J.

Furthermore, as shown in FIG 13, the superimposed image generation section 12 may generate a superimposed image PW1D having the axial image PA including the position of the distal end portion 2C. In this case, the superimposed image generation section 12 may display a distal end portion display mark P2CD indicating not only the position of the distal end portion 2C but also the direction of the distal end portion 2C, or may display only the direction. That is, the distal end portion display mark achieves a predetermined effect if the distal end portion display mark indicates at least one of the position and the direction of the distal end portion. In addition, the superimposed image generation section 12 may display the roll angle of the distal end portion 2C by the distal end portion display mark.

Note that, in FIG 13, the superimposed image generation section 12 does not display the route image PR1 of the route from the pharynx portion 7A to the distal end portion 2C, which is the insertion route through which the distal end portion has already passed, which provides an excellent visibility of the superimposed image.

In addition, the tomographic image generation section 14 may generate the coronal image PC or the sagital image PS, which includes the position of the distal end portion 2C.

That is, the tomographic image generation section 14 generates a tomographic image based on the position and the direction of the distal end portion 2C, but is capable of generating a tomographic image based only on the position of the distal end portion 2C.

### <Step S16> Superimposed Display

The superimposed image PW1 generated by the superimposed image generation section 12 is displayed on the display section 4 together with the real image and the VBS image.

Note that the superimposed image PW1 may be constantly displayed on the navigation screen, may be brought temporarily into a non-display state by a setting by the operator, or may be brought automatically into the non-display state under the control by the control section 10. In addition, the kind of the tomographic images displayed in the superimposed image PW1 may be changed by the setting by the operator or under the control by the control section 10.

The image to be displayed on the navigation screen may be selected based on the position of the distal end portion 2C. For example, when the distal end portion 2C is brought near to the bifurcation portion J, the display mode may be set to a display mode for displaying the navigation screen including the superimposed image PW1, and after the distal end portion 2C passed through the bifurcation portion J, the display mode may be switched to a display mode for displaying the navigation screen on which the superimposed image PW1 is not displayed.

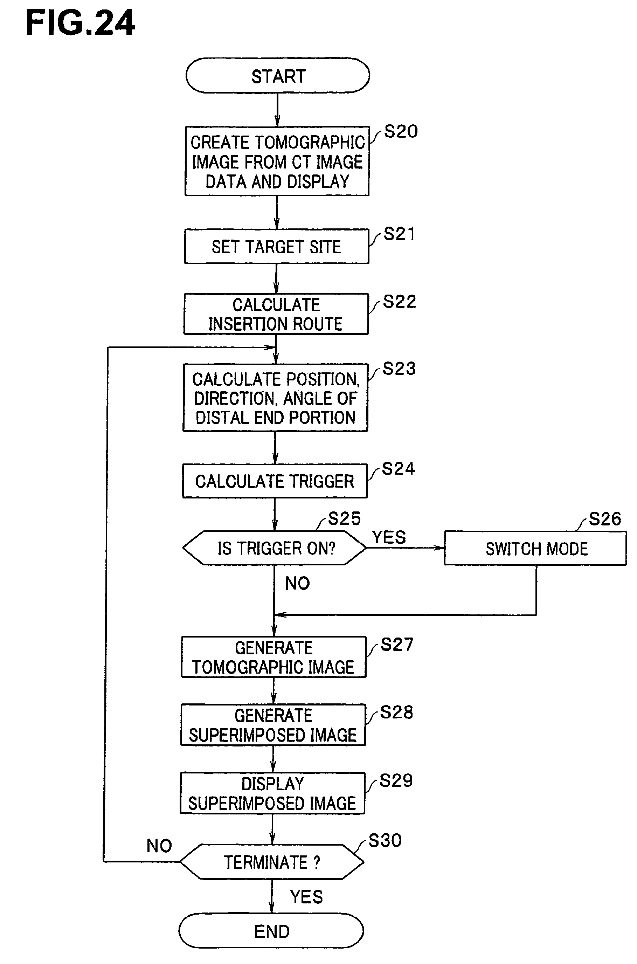

The switching of the display mode is controlled by the control section 10 depending on presence or absence of setting of a trigger, similarly in the switching of the navigation mode (See FIG 24).

### <Step S 17> Terminate?

Until the distal end portion 2C is inserted close to the target site 9G (S17: Yes), the processing steps in the step S 13 and after are repeatedly performed.

When the distal end portion 2C is inserted close to the target site 9G, the insertion navigation mode is terminated, and the treatment instrument 6 is protruded from the distal end portion 2C, and biopsy or the like is performed on the target site 9G

As described above, in the medical device 1, the operator can easily recognize the position of the distal end portion 2C based on the superimposed image displayed on the display section 4. Furthermore, the operator can recognize the state of the tissues in the vicinity of the distal end portion 2C based on the tomographic image PO. Therefore, the medical device 1 facilitates the insertion of the distal end portion 2C of the insertion portion 2A to the target site 9G

### <Second Embodiment>

Hereinafter, a medical device 1 A according to the second embodiment of the present invention will be described with reference to the drawings. The medical device 1A is similar to the medical device 1. Therefore, the same constituent elements are attached with the same reference numerals and the description thereof will be omitted.

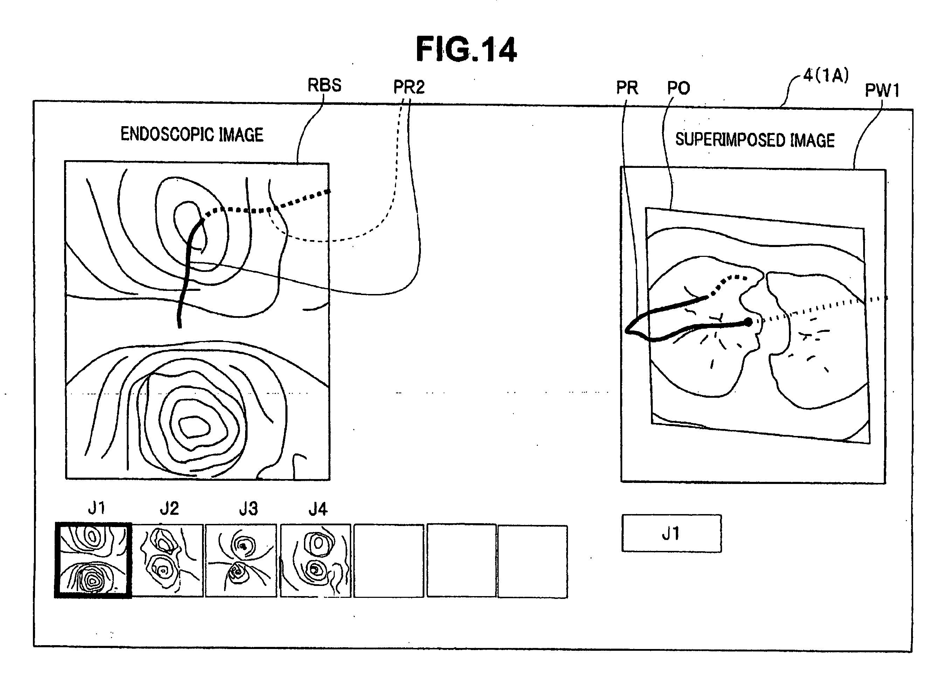

As shown in FIG 14, in the medical device 1A, the VBS image is not displayed on the navigation screen, and a second route image PR2 indicating the insertion route R is displayed on the real image in a superimposed manner.

In order to insert the second route image PR2 in the real image, the second route image PR2 to be superimposed on the VBS image corresponding to the real image is generated, and the generated second route image PR2 is superimposed on the real image.

The operator can perform insertion operation while checking the insertion route R with reference to the second route image PR2 which is displayed on the real image in a superimposed manner and recognizing the position and the like of the distal end portion 2C with reference to the superimposed image PW1.

The medical device 1A has the same effects as those of the medical device 1, and further includes an advantage of a simple navigation screen with improved visibility. Note that the various kinds of configurations described in the medical device 1 can be also used in the medical device 1A, and the configuration of the medical device 1A can be also used in the medical device 1.

### <Modified Examples 1 and 2 of Second Embodiment>

Hereinafter, description will be made on a medical device 1B according to the modified example 1 of the second embodiment of the present invention, and a medical device 1C according to the modified example 2 of the second embodiment of the present invention. The medical devices 1B and 1C are similar to the medical device 1A. Therefore, the same constituent elements are attached with the same reference numerals and description thereof will be omitted.

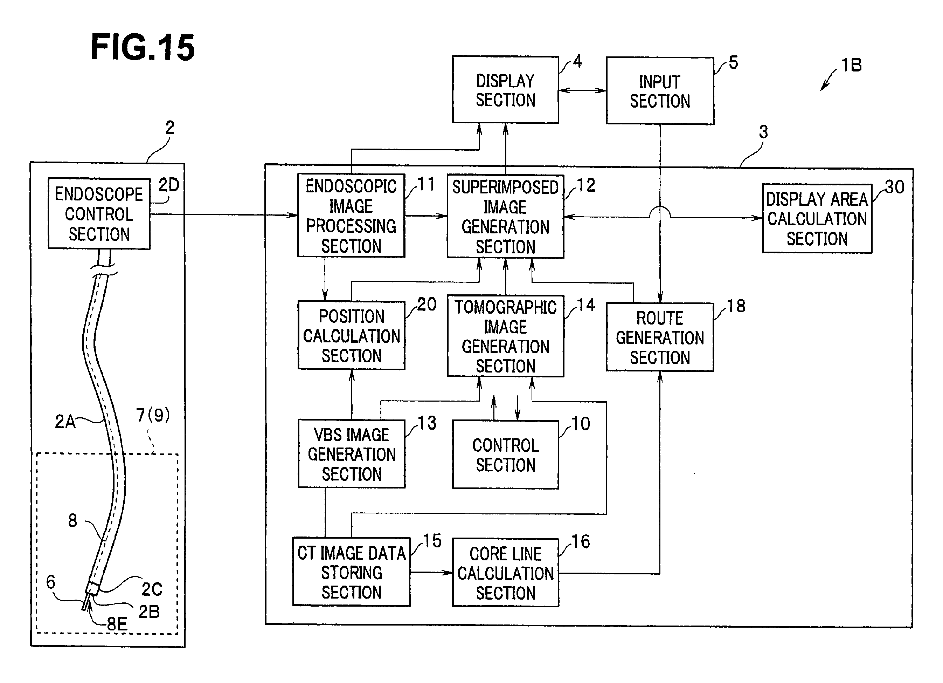

As shown in FIG 15, the medical device 1B includes a display area calculation section 30 as display area calculation means. The display area calculation section 30 calculates the display area of the second route image PR2 displayed on the VBS image in a superimposed manner.

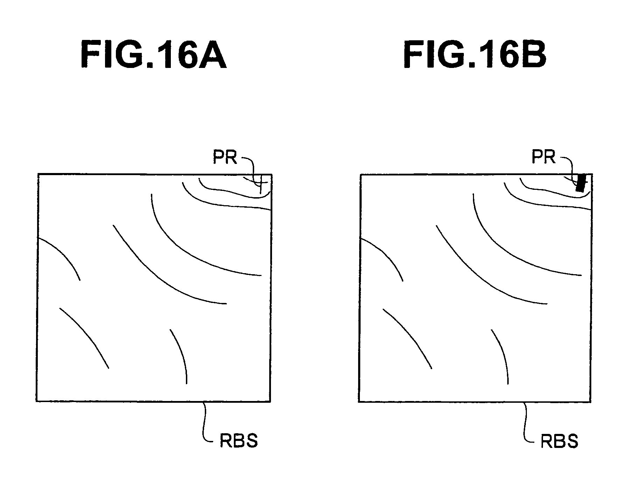

The insertion route R is calculated along the core line S as a center of the bronchus 9 having a predetermined thickness. Therefore, as shown in FIG 16A, the second route displayed on the VBS image is short depending on the direction and the like of the distal end portion 2C. In such a case, it is not easy for the operator to recognize a correct insertion route R.

However, as shown in FIG 16B, in the medical device 1B, when the display area calculated by the display area calculation section 30 is equal to or smaller than a first predetermined value, the superimposed image generation section 12 highlights the second route image PR2.

For example, the display area calculation section 30 counts the number K of the pixels of the second route image PR2 in the VBS image composed of 500 ×500 pixels. Then, when the number of K of the pixels is equal to or smaller than the first predetermined value K1, the superimposed image generation section 12 displays a line representing the route image PR in a thick manner such that the number of the pixels becomes K1, for example. That is, the shorter the route displayed in a superimposed manner is, the thicker the displayed route image PR is.

In addition, when halation or the like occurs in the real image, the real image partly becomes stark white in some cases, and in other cases, the color inside the lumen and the color of the second route image PR2 are hard to be distinguished from each other. Therefore, as a method of highlighted display of the second route image PR2, the color or the type of the line may be changed, or the second route image may be displayed in a blinking manner.

Alternatively, the display area calculation section 30 may calculate the average luminance not for the pixels in the whole of the real image RBS but for the pixels within a range of a predetermined region of interest (ROI), and may change the display method so as to improve the visibility of the second route image PR2 depending on the change of the average luminance.

It is preferable to set the ROI within a range surrounding the second route image PR2, and the shape of the ROI may be any of a circle, an ellipse, a rectangular, a square, and the like. In addition, the shape is not limited to a preset shape, and a figure whose range surrounding the second route image PR2 has the minimum area may be selected for each processing, or a previously selected figure may be enlarged or reduced so as to cover the range surrounding the second route image PR2.

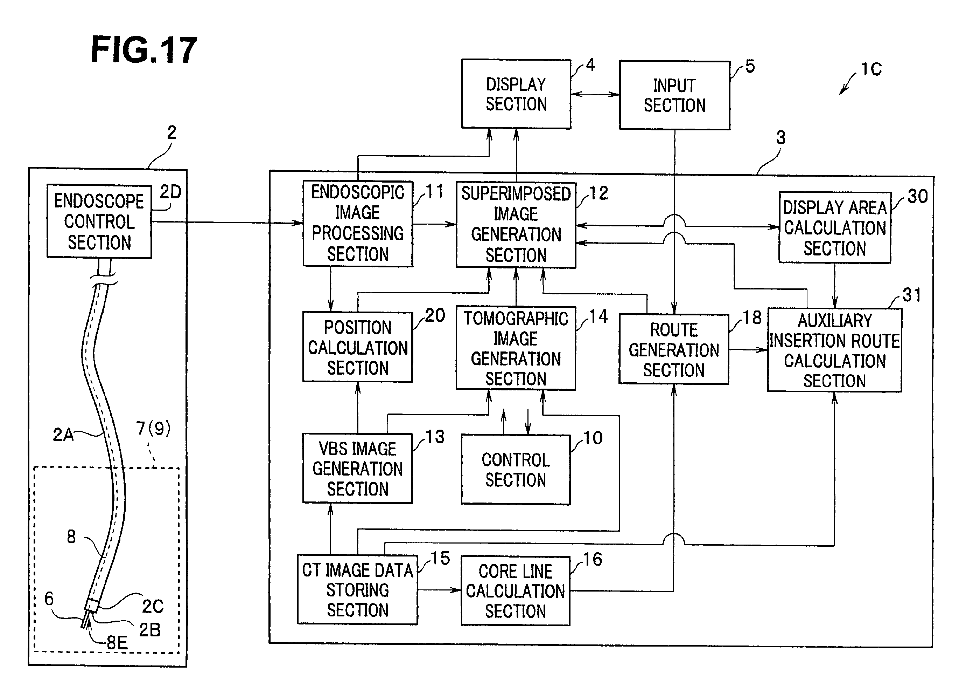

On the other hand, as shown in FIG 17, the medical device 1C according to the modified example 2 of the second embodiment includes an auxiliary insertion route generation section 31 as auxiliary insertion route generation means.

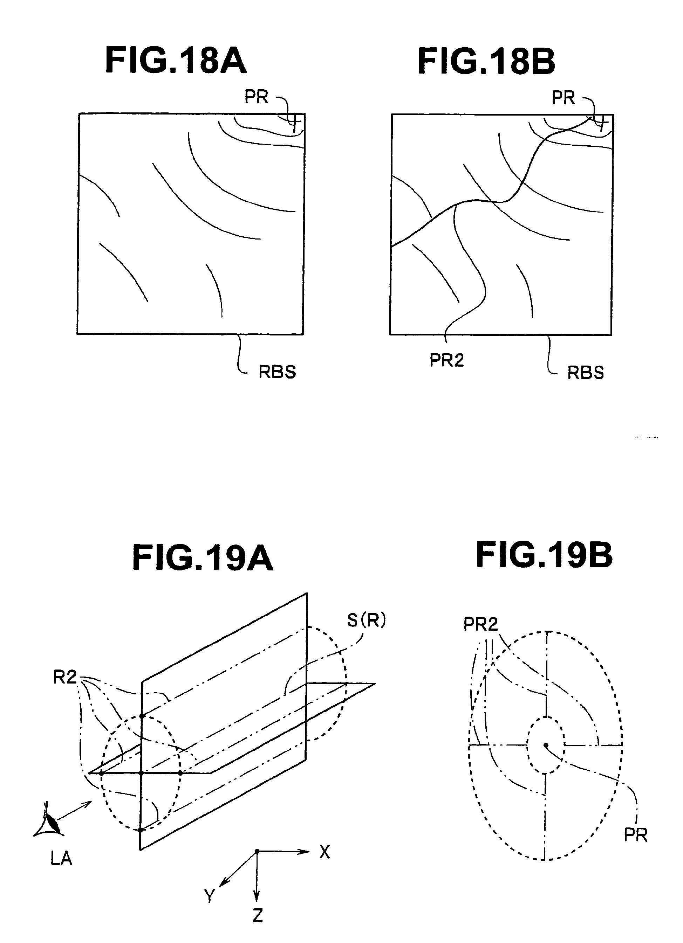

As shown in FIG 18A, depending on the position or the direction of the distal end portion 2C, the displayed second route image PR2 is extremely short in some cases, or the second route image is not displayed at all in other cases. In such cases, it is not easy for the operator to recognize a correct insertion route.

However, as shown in FIG 18B, in the medical device 1C, when the display area of the second route image PR2 calculated by the display area calculation section 30 is equal to or smaller than a second predetermined value K2, the superimposed image generation section 12 displays an auxiliary insertion route image PSR instead of the second route image PR2, or together with the second route image PR2 in a superimposed manner. In this case, the second predetermined value K2 may be zero, for example.

The auxiliary insertion route generation section 31 uses not only core line information but also volume information as three-dimensional shape information of the lumen. As already described above, the core line S is a line connecting the gravity center points on a vertical plane in the tract direction of the lumen, and the volume information is information indicating a position of the luminal wall of the lumen.

That is, as shown in FIG 19A, the auxiliary insertion route generation section 31 generates an auxiliary insertion route SR which is a cross line between the plane including the insertion route R and the luminal wall of the bronchus 9 as volume information. Note that FIG 19A shows a case where the lumen is a straight duct, for illustrative purpose. Accordingly, the plane including the insertion route R is a two-dimensional plane. However, the actual lumen is curved, so that the plane including the insertion route R is also a curved plane.

FIG 19A shows a case where four auxiliary insertion routes SR are generated by two planes including the insertion route R and perpendicular to each other. Therefore, even in a case where the direction of the core line S and the direction of the line of sight LA are coincident with each other, the four auxiliary insertion route images PSR are superimposed on the endoscopic image, as shown in FIG 19B.

Note that the auxiliary insertion route generation section 31 may generate more than four, for example, eight auxiliary insertion routes SR.

The medical devices 1B and 1C have the same effects as those of the medical devices 1 and 1A, and further have an advantage of excellent visibility of the insertion route R on the navigation screen. Note that the various kinds of configurations described in the medical devices 1 and 1A can be also used in the medical devices 1B and 1C, and the configurations of the medical devices 1B and 1C can be also used in the medical devices 1 and 1A.

### < Third Embodiment>

Hereinafter, description will be made on the medical device 1D according to the third embodiment of the present invention with reference to drawings. The medical device 1D is similar to the medical device 1. Therefore, the same constituent elements are attached with the same reference numerals and description thereof will be omitted.

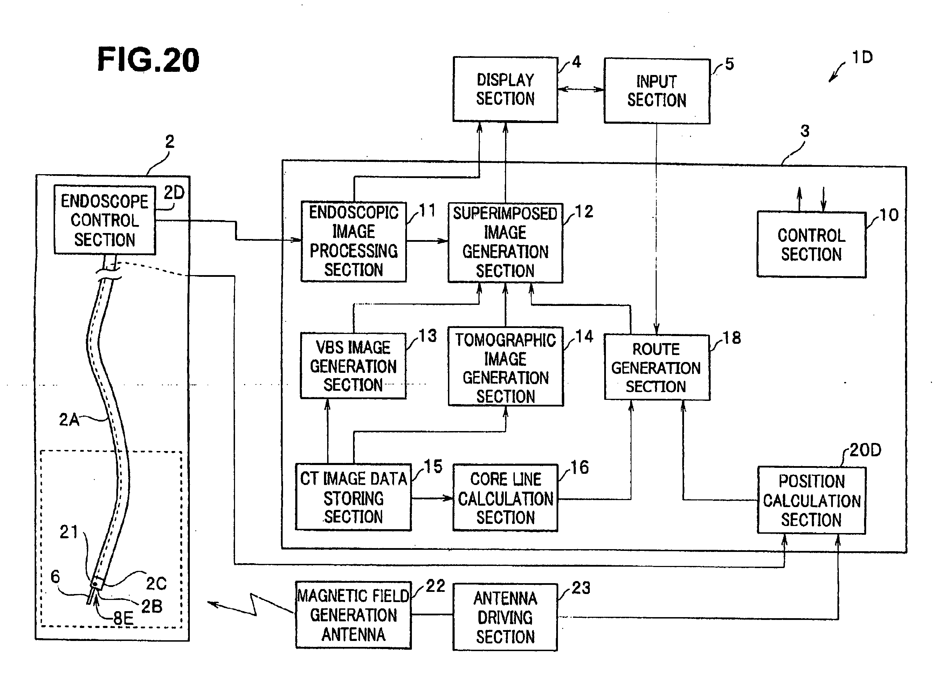

As shown in FIG 20, a magnetic field sensor 21 as a position sensor is disposed at a distal end portion 2C of an insertion portion 2A of the medical device 1D, and a position calculation section 20D calculates the position, the direction, and the roll angle of the distal end portion 2C based on the data acquired by the magnetic field sensor 21.

The magnetic field sensor detects the magnetic field generated by a plurality of magnetic field generation antennae 22 disposed outside the subject 7, and thereby the position calculation section 20D detects the position and the like of the distal end portion 2C. That is, the disposed positions of the magnetic field sensor 21 and the image pickup section 2B which are disposed at the distal end portion 2C are well-known. Therefore, the position calculation section 20D detects the position and the direction of line of sight, and the roll angle of the image pickup section 2B. Note that, as the magnetic field detection sensor, an MR sensor, a Hall element, a coil and the like can be used.

The medical device 1D has the same effects as those of the medical device 1. Note that the various kinds of configurations described in the medical devices 1, and 1A to 1C can be also used in the medical device 1D, and the configuration of the medical device 1D can be also used in the medical devices, 1 and 1A to 1C.

### <Modified Example of Third Embodiment>

Hereinafter, description will be made on a medical device 1DA according to a modified example of the third embodiment of the present invention with reference to drawings. The medical device 1DA is similar to the medical device 1D. Therefore, the same constituent elements are attached with the same reference numerals and description thereof will be omitted.

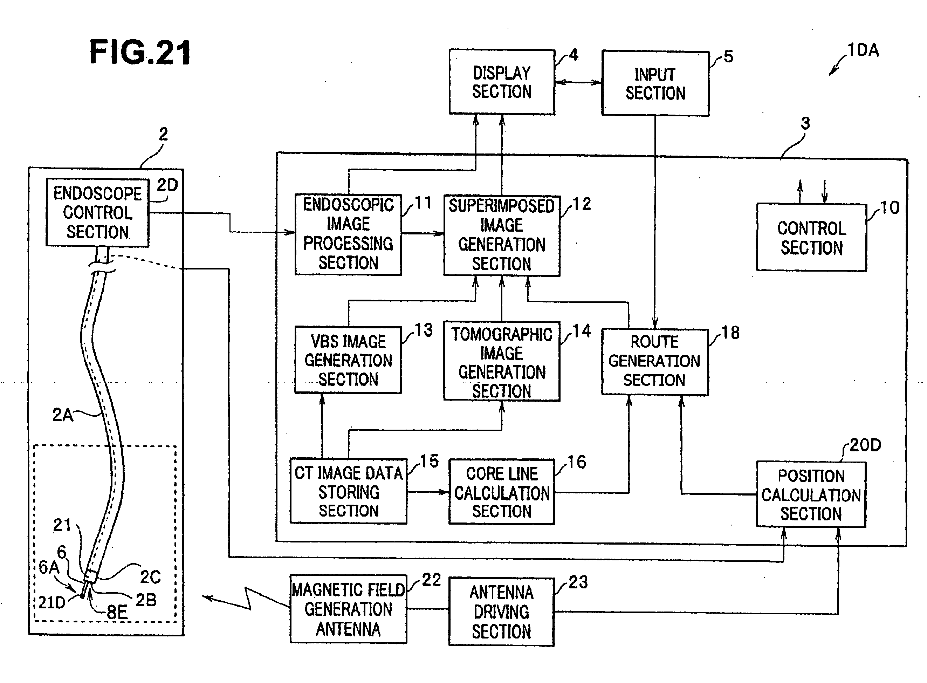

As shown in FIG 21, a magnetic field sensor 21D as a position sensor is disposed at a treatment instrument distal end portion 6A of the treatment instrument 6 of the medical device 1DA. During the insertion operation of the insertion portion 2A, the treatment instrument distal end portion 6A is housed in the distal end portion 2C of the insertion portion 2A. Therefore, the position calculation section 20D calculates the position, the direction and the roll angle of the distal end portion 2C based on the data acquired by the magnetic field sensor 21D. Furthermore, during the insertion operation of the treatment instrument 6, the position calculation section calculates the position, the direction, and the roll angle of the treatment instrument distal end portion 6A. Note that the treatment instrument distal end portion 6A is an edge when the treatment instrument is a needle, but may be a center of cups when the treatment instrument is a biopsy forceps, and may be a center of brush when the treatment instrument is a brush.

Note that, in order to support the insertion into the bifurcations of the bronchus, when a guiding instrument as a forceps which can bend a taper-shaped distal end portion having joints by manual operation is used as the treatment instrument 6, the magnetic field sensor 21D may be disposed at the distal end portion of the guiding instrument.

The medical device 1DA has the same effects as those of the medical device 1D, and moreover can acquire position information of the treatment instrument distal end portion 6A protruded from the channel opening 8E.

### <Fourth Embodiment>

Hereinafter, description will be made on a medical device 1E according to the fourth embodiment of the present invention, with reference to drawings. The medical device 1E is similar to the medical device 1. Therefore, the same constituent elements are attached with the same reference numerals and description thereof will be omitted.

In the medical device 1E, when the distal end portion 2C of the insertion portion 2A reaches in the vicinity of the target site 9G, the tomographic image displayed on the navigation screen is changed. In other words, the image displayed by the display section 4 is selected based on the position of the distal end portion 2C by the control section 10. More specifically, when the distance between the position of the distal end portion 2C and the position of the target site 9G is equal to or smaller than a predetermined value, or the distal end portion 2C passed through the last bifurcation portion, the navigation mode is switched from an insertion portion insertion-supporting mode to a treatment instrument operation supporting mode. It is needless to say that the operator may select the navigation mode.

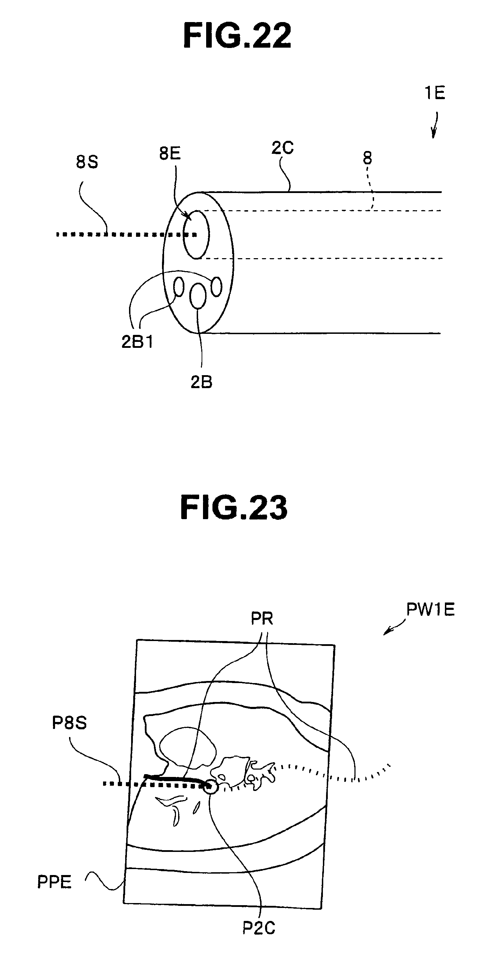

In this embodiment, as shown in FIG. 22, the distal end portion 2C of the medical device 1E is provided with an image pickup section 2B and an illumination section 2B1, and the treatment instrument 6 can be protruded from the channel opening 8E. The position of the channel opening 8E is different from the position of the image pickup section 2B. In order to perform more precise treatment instrument operation support, in the medical device 1E, it is preferable to use the position of the channel opening 8E as the position of the distal end portion 2C, when the navigation mode is switched to the treatment instrument operation supporting mode.

Then, the tomographic image generation section 14 generates a tomographic image PPE of a plane including the position of the channel opening 8E and parallel to the axis direction of the channel 8, that is, the plane parallel to the direction of the distal end portion 2C. Furthermore, as shown in FIG 23, the superimposed image generation section 12 generates a superimposed image PW1E in which the extended line P8S of the channel 8 is displayed on the tomographic image PPE in a superimposed manner. The extended line 8S shows a direction in which the treatment instrument 6 is protruded from the channel opening 8E.

The extended line P8S may be calibrated or the color of the line may be changed depending on the length. Furthermore, the direction of the extended line P8S may have a predetermined angle with respect to the direction of the distal end portion 2C, and the angle may be arbitrarily changed by the operator.

Hereinafter, with reference to the flowchart in FIG 24, the switching of the navigation mode in the medical device 1E will be described.

### <Step S20 - S23>

The processings in these steps are similar to those in the steps S 10 to S 13 in the medical device 1 according to the first embodiment which was described with reference to FIG 4.

### <Step S24 > Trigger Calculation

In the medical device 1E, a trigger is set by the control section 10 depending on the position of the distal end portion 2C calculated in the step S21. For example, the trigger is set when the distance between the position of the distal end portion 2C and the target site 9G is equal to or smaller than the predetermined value. In this case, the distance between the position of the distal end portion 2C and the target site 9G may be a direct distance, or a distance of the insertion route via the core line S.

In addition, the trigger is set when the inner diameter of the portion of the bronchus 9 at which the distal end portion 2C is positioned is equal to or smaller than a predetermined value, or when the difference between the inner diameter of the portion of the bronchus 9 at which the distal end portion 2C is positioned and the outer diameter of the insertion portion 2A is equal to or smaller than a predetermined value, for example.

Furthermore, the trigger may be set not only automatically by the control section 10 but also by the setting operation by the operator using the input section 5. Alternatively, the trigger may be set when detecting that the image of the treatment instrument 6 is reflected on the real image, that is, the operator has started biopsy by protruding the treatment instrument 6 from the channel opening 8E.

For example, when the treatment instrument 6 is protruded from the channel opening 8E, the luminance of the pixels within a predetermined region of interest (ROI) in the real image is increased. Therefore, the average luminance in the ROI is calculated and the trigger may be set depending on the change of the average luminance.

### <Step S25> Is Trigger ON? <Step S26> Mode Switching

When the trigger is ON (YES), the navigation mode is switched in step S26. In contrast, when the trigger is OFF (NO), the current navigation mode is continued.

### <Step S27 to S30>

The processings in these steps are similar to those in the steps S 14 to S 17 in the medical device 1 according to the first embodiment which was described with reference to FIG 4.

The medical device 1E has the same effects as those in the medical device 1, and further performs treatment instrument operation support also after the distal end portion 2C is inserted close to the target site 9G Note that the various kinds of configurations described in the medical device 1, and 1A to 1D can be also used in the medical device 1E, and the configuration of the medical device 1E can be also used in the medical device 1, and 1A to 1D.

### <Fifth Embodiment>

Hereinafter, a medical device 1F according to the fifth embodiment of the present invention will be described with reference to drawings. The medical device 1F is similar to the medical device 1. Therefore, the same constituent elements are attached with the same reference numerals and description thereof will be omitted.

As shown in FIG 24, an endoscope apparatus 2F in the medical device 1F includes at the distal end portion 2C a convex scanning type ultrasound transducer 40 which scans an arc-shaped range. The operator can confirm the positions of lymph nodes, blood vessels, and the like, based on the ultrasound image.

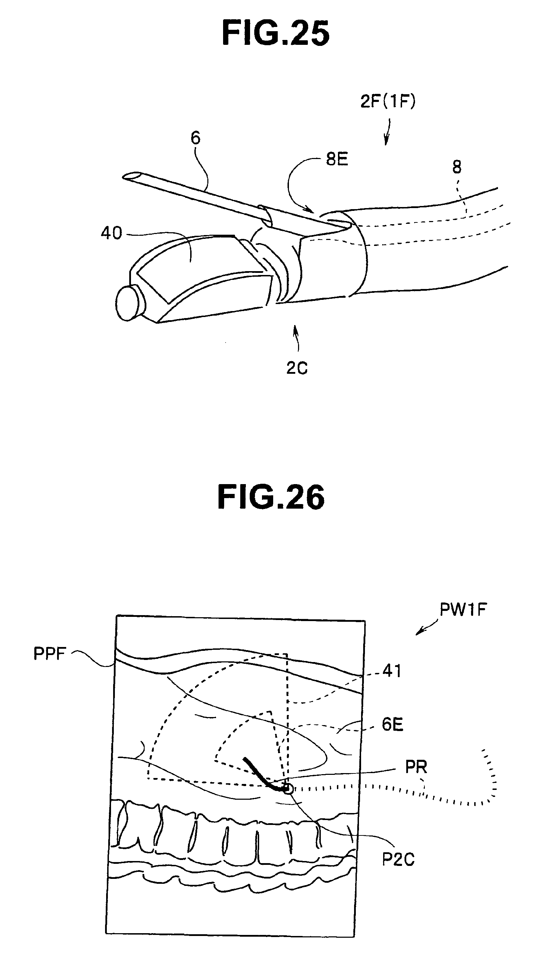

Then, when the distal end portion 2C is inserted close to the target site 9G, the navigation mode is switched to the treatment instrument operation supporting mode, and the tomographic image generation section 14 generates a tomographic image PPF (see FIG 26) of the scanning plane of the ultrasound transducer 40, which includes the position of the distal end portion 2C.

Furthermore, as shown in FIG 25, in the treatment instrument operation supporting mode, the superimposed image generation section 12 generates a superimposed image PW1F in which a scanning range 41 of the ultrasound transducer 40 and the range 6E which can be treated by the treatment instrument 6 protruded from the channel opening 8E are displayed on the tomographic image PPF in a superimposed manner.

The operator can recognize the three-dimensional relation between the scanning range 41 and the treatable range 6E, by changing the position of the line of sight when viewing the superimposed image PW1F which is a three-dimensional model image.

Note that, also in the medical device 1F, the switching of the navigation mode is performed by detecting the setting of the trigger, similarly as in the medical device 1E according to the fourth embodiment.

The medical device 1F has the same effects as those in the medical devices 1 and the like, and further performs treatment instrument operation support also after the distal end portion 2C is inserted close to the target site 9G Note that various kinds of configurations described in the medical devices 1, and 1A to 1E can be also used in the medical device 1F, and the configuration of the medical device 1F can be also used in the medical devices 1, and 1A to 1E.

Note that the medical devices in the above-described embodiments can be also used when observing the whole of the lumen, that is, when performing a screening without determining a target site, for example. In such a case, a trajectory of the endoscope distal end is displayed instead of the insertion route. The points configuring the trajectory may be positions calculated by the position calculation means or may be points on the center line of the luminal organ located in the vicinity of the calculated positions. In addition, the trajectory to be displayed may be a history of movement which shows the whole previous movement of the endoscope distal end or may be just a trajectory during a predetermined time period or a trajectory within a predetermined space. Furthermore, the center line of the luminal organ is displayed on the trajectory in a superimposed manner, thereby allowing the operator to easily determine that which part of the luminal organ has been observed.

In a case where a screening is performed, also when the endoscope distal end is inserted to a predetermined site and thereafter the endoscope is extracted to reach the carina, for example, the trajectory representing the whole previous movement of the endoscope distal end may be displayed. At that time, it is preferable to make the trajectory distinguishable by displaying the line indicating the position of the endoscope distal end on the deeper side than the carina, that is, the trajectory, in a different color, or with a dotted line.

Thus, the present invention is not limited to the foregoing embodiments and modified examples and various changes and modifications are possible. For example, as already explained above, the foregoing embodiments and modified examples may be appropriately combined.

The present application is filed claiming the priority of Japanese Patent Application No. 2011-012103 filed in Japan on January 24, 2011.

## Claims

1. A medical device (1, 1A, 1B, 1C, 1D, 1DA, 1E, 1F) comprising:
storing means (15) configured to store previously acquired three-dimensional image data of a subject (7);
position calculation means (20, 20D) configured to calculate a position and a direction of a distal end portion (2C) of insertion means (2A) of an endoscope apparatus (2) inserted into a lumen in a body of the subject (7);
route generation means (18) configured to generate a three-dimensional insertion route (R) for inserting the distal end portion (2C) to a target position (9G) through the lumen in the body of the subject (7), based on the three-dimensional image data;
**characterized in that** the medical device (1, 1A, 1B, 1C, 1D, 1DA, 1E, 1F) further comprises tomographic image generation means (14) configured to generate a two-dimensional tomographic image (PA, PC, PS, PO) based on the position and the direction of the distal end portion (2C), from the three-dimensional image data; and
superimposed image generation means (12) configured to generate a superimposed image (PW1) on which the two-dimensional tomographic image (PA, PC, PS, PO) and the three-dimensional insertion route (R) are displayed in a superimposed manner as a three-dimensional model image viewed along an arbitrary line of sight (LA) set by an operator.

2. The medical device according to claim 1, **characterized in that** the tomographic image generation means (14) generates a tomographic image (PA, PC, PS, PO) on a plane which includes a position of the distal end portion (2C) and which is perpendicular to a direction of the distal end portion (2C).

3. The medical device according to claim 1, **characterized in that** the superimposed image generation means superimposes a distal end portion display mark (P2C) indicating at least one of the position and the direction of the distal end portion (2C) on the three-dimensional model image.

4. The medical device according to claim 1, **characterized in that** the superimposed image generation means superimposes on the insertion route (R) a bifurcation portion display mark (PJ1, PJ2, PJ3, PJ4) indicating a position of a bifurcation portion of the lumen which is branched off.

5. The medical device according to claim 1, **characterized in that** the superimposed image generation means displays the insertion route (R) such that a part of the insertion route (R) from an insertion start position to the position of the distal end portion (2C) is distinguishable from a part of the insertion route (R) from the position of the distal end portion (2C) to the target position (9G).

6. The medical device according to claim 1, **characterized in that** the superimposed image generation means does not display the part of the insertion route (R) from the insertion start position to the position of the distal end portion (2C).

7. The medical device according to claim 1, **characterized in that** it further comprises:
image pickup means (2B) configured to pick up an endoscopic image (RBS), the image pickup means (2B) being provided at the distal end portion (2C) of the insertion means (2A);
virtual endoscopic image generation means (13) configured to generate a virtual endoscopic image using a position, a direction, and a roll angle of the distal end portion (2C) as line-of-sight parameters, from the three-dimensional image data;
and
display means (4) which displays at least one of the three-dimensional model image generated by the superimposed image generation means, the endoscopic image, and the virtual endoscopic image.

8. The medical device according to claim 7, **characterized in that** the position calculation means (20, 20D) calculates the position and the direction of the distal end portion (2C) based on the line-of-sight parameters of the virtual endoscopic image which is similar to the endoscopic image.

9. The medical device according to claim 8, **characterized in that** the superimposed image generation means displays a second insertion route image (PR2) indicating the insertion route in a superimposed manner on at least one of the endoscopic image and the virtual endoscopic image.

10. The medical device according to claim 9, **characterized in that** it further comprises,
display area calculation means (30) configured to calculate a display area of the second insertion route image (PR2) which is displayed in the superimposed manner,
wherein when the display area is equal to or smaller than a first predetermined value, the superimposed image generation means displays the second insertion route image (PR2) in a highlighted manner.

11. The medical device according to claim 9, **characterized in that** it further comprises:
display area calculation means (30) configured to calculate a display area of the second insertion route image (PR2) which is displayed in the superimposed manner; and
auxiliary insertion route calculation means (31) configured to calculate an auxiliary insertion route (SR) which is a cross line between a plane including the insertion route and a luminal wall of the lumen,
wherein when the display area is equal to or smaller than a second predetermined value, the superimposed image generation means displays an auxiliary route image (PSR) indicating the auxiliary insertion route (SR) in a superimposed manner.

12. The medical device according to claim 9, **characterized in that** the three-dimensional model image generated by the superimposed image generation means is selected based on the position of the distal end portion (2C).

13. The medical device according to claim 12, **characterized in that** the tomographic image generation means (14) further generates a tomographic image (PA, PC, PS, PO) on a plane which includes the position of the distal end portion (2C) and which is parallel to the direction of the distal end portion (2C).

## Patentansprüche

1. Medizinische Vorrichtung (1, 1A, 1B, 1C, 1D, 1DA, 1E, 1F), umfassend:
ein Speichermittel (15), welches dazu eingerichtet ist, vorab von einem Subjekt (7) gewonnene dreidimensionale Bilddaten zu speichern;
ein Positionsberechnungsmittel (20, 20D), welches dazu eingerichtet ist, eine Position und eine Ausrichtung eines in ein Lumen eines Körpers des Subjekts (7) eingeführten distalen Endabschnitts (2C) eines Einführmittels (2A) eines Endoskopgeräts (2) zu berechnen;
ein Pfaderzeugungsmittel (18), welches dazu eingerichtet ist, basierend auf den dreidimensionalen Bilddaten einen dreidimensionalen Einführpfad (R) zum Einführen des distalen Endabschnitts (2C) durch das Lumen in dem Körper des Subjekts (7) zu einer Zielposition (9G) zu erzeugen;
**dadurch gekennzeichnet, dass** die medizinische Vorrichtung (1, 1A, 1B, 1C, 1D, 1DA, 1E, 1F) ferner ein Tomographiebild-Erzeugungsmittel (14) umfasst, welches dazu eingerichtet ist, basierend auf der Position und der Ausrichtung des distalen Endabschnitts (2C) aus den dreidimensionalen Bilddaten ein zweidimensionales Tomographiebild (PA, PC, PS, PO) zu erzeugen; und
ein Überlagerungsbild-Erzeugungsmittel (12), welches dazu eingerichtet ist, ein Überlagerungsbild (PW1) zu erzeugen, auf dem das zweidimensionale Tomographiebild (PA, PC, PS, PO) und der dreidimensionale Einführpfad (R) als ein entlang einer beliebigen, von einem Benutzer vorgegebenen Sichtachse (LA) betrachtetes dreidimensionales Modellbild auf überlagerte Weise angezeigt sind.

2. Medizinische Vorrichtung nach Anspruch 1,
**dadurch gekennzeichnet, dass** das Tomographiebild-Erzeugungsmittel (14) ein Tomographiebild (PA, PC, PS, PO) auf einer Ebene erzeugt, die eine Position des distalen Endabschnitts (2C) enthält und die senkrecht zu einer Ausrichtung des distalen Endabschnitts (2C) verläuft.

3. Medizinische Vorrichtung nach Anspruch 1,
**dadurch gekennzeichnet, dass** das Überlagerungsbild-Erzeugungsmittel dem dreidimensionalen Modellbild eine Anzeigemarkierung (P2C) für den distalen Endabschnitt überlagert, die die Position und/oder die Ausrichtung des distalen Endabschnitts (2C) angibt.

4. Medizinische Vorrichtung nach Anspruch 1,
**dadurch gekennzeichnet, dass** das Überlagerungsbild-Erzeugungsmittel auf dem Einführpfad (R) eine Verzweigungsabschnitt-Anzeigemarkierung (PJ1, PJ2, PJ3, PJ4) überlagert, die eine Position eines Verzweigungsabschnitts des sich abzweigenden Lumens angibt.

5. Medizinische Vorrichtung nach Anspruch 1,
**dadurch gekennzeichnet, dass** das Überlagerungsbild-Erzeugungsmittel den Einführpfad (R) dergestalt anzeigt, dass ein Teil des Einführpfads (R) von einer Einführungsanfangsposition zu der Position des distalen Endabschnitts (2C) gegenüber einem Teil des Einführpfads (R) von der Position des distalen Endabschnitts (2C) zu der Zielposition (9G) unterscheidbar ist.

6. Medizinische Vorrichtung nach Anspruch 1,
**dadurch gekennzeichnet, dass** das Überlagerungsbild-Erzeugungsmittel den Teil des Einführpfads (R) von der Einführungsanfangsposition zu der Position des distalen Endabschnitts (2C) nicht anzeigt.

7. Medizinische Vorrichtung nach Anspruch 1,
**dadurch gekennzeichnet, dass** sie ferner umfasst:
ein Bildaufnahmemittel (2B), welches dazu eingerichtet ist, ein Endoskopiebild (RBS) aufzunehmen, wobei das Bildaufnahmemittel (2B) an dem distalen Endabschnitt (2C) des Einführmittels (2A) vorgesehen ist;
ein Erzeugungsmittel (13) für ein virtuelles Endoskopiebild, welches dazu eingerichtet ist, unter Verwendung einer Position, einer Ausrichtung und eines Rollwinkels des distalen Endabschnitts (2C) als Sichtachsenparameter aus den dreidimensionalen Bilddaten ein virtuelles Endoskopiebild zu erzeugen; und
ein Anzeigemittel (4), welches zumindest das mit dem Überlagerungsbild-Erzeugungsmittel erzeugte dreidimensionale Modellbild und/oder das Endoskopiebild und/oder das virtuelle Endoskopiebild anzeigt.

8. Medizinische Vorrichtung nach Anspruch 7,
**dadurch gekennzeichnet, dass** das Positionsberechnungsmittel (20, 20D) die Position und die Ausrichtung des distalen Endabschnitts (2C) basierend auf den Sichtachsenparametern des virtuellen Endoskopiebilds berechnet, welches dem Endoskopiebild ähnelt.

9. Medizinische Vorrichtung nach Anspruch 8,
**dadurch gekennzeichnet, dass** das Überlagerungsbild-Erzeugungsmittel eine Abbildung (PR2) eines zweiten Einführpfads anzeigt, die den Einführpfad auf überlagerte Weise auf dem Endoskopiebild und/oder dem virtuellen Endoskopiebild angibt.

10. Medizinische Vorrichtung nach Anspruch 9,
**dadurch gekennzeichnet**, das sie ferner umfasst:
ein Anzeigebereich-Berechnungsmittel (30), welches dazu eingerichtet ist, einen Anzeigebereich der Abbildung (PR2) des zweiten Einführpfads zu berechnen, welche auf überlagerte Weise angezeigt ist,
wobei das Überlagerungsbild-Erzeugungsmittel die Abbildung (PR2) des zweiten Einführpfads auf hervorgehobene Weise anzeigt, wenn der Anzeigebereich kleiner oder gleich einem vorbestimmten Wert ist.

11. Medizinische Vorrichtung nach Anspruch 9,
**dadurch gekennzeichnet, dass** sie ferner umfasst:
ein Anzeigebereich-Berechnungsmittel (30), welches dazu eingerichtet ist, einen Anzeigebereich der Abbildung (PR2) des zweiten Einführpfads zu berechnen, welche auf überlagerte Weise angezeigt ist; und
ein Berechnungsmittel (31) für einen zusätzlichen Einführpfad (SR), welches dazu eingerichtet ist, einen zusätzlichen Einführpfad zu berechnen, der eine Schnittlinie zwischen einer den Einführpfad enthaltenden Ebene und einer Lumenwand des Lumens ist,
wobei das Überlagerungsbild-Erzeugungsmittel eine Zusatzpfadabbildung (PSR) anzeigt, die den zusätzlichen Einführpfad (SR) auf überlagerte Weise angibt, wenn der Anzeigebereich kleiner oder gleich einem zweiten vorbestimmten Wert ist.

12. Medizinische Vorrichtung nach Anspruch 9, **dadurch gekennzeichnet, dass** das von dem Überlagerungsbild-Erzeugungsmittel erzeugte dreidimensionale Modellbild basierend auf der Position des distalen Endabschnitts (2C) ausgewählt ist.

13. Medizinische Vorrichtung nach Anspruch 12,
**dadurch gekennzeichnet, dass** das Tomographiebild-Erzeugungsmittel (14) außerdem ein Tomographiebild (PA, PC, PS, PO) auf einer Ebene erzeugt, die die Position des distalen Endabschnitts (2C) enthält und die parallel zu der Ausrichtung des distalen Endabschnitts (2C) verläuft.

## Revendications

1. Dispositif médical (1, 1A, 1B, 1C, 1D, 1DA, 1E, 1F) comprenant :
un moyen (15) de stockage configuré pour stocker des données d'images en trois dimensions acquises au préalable d'un sujet (7) ;
un moyen (20, 20D) de calcul de position configuré pour calculer une position et une direction d'une partie d'extrémité distale (2C) d'un moyen (2A) d'insertion d'un appareil d'endoscope (2) inséré dans une lumière dans un corps du sujet (7) ;
un moyen (18) de génération de voie configuré pour générer une voie (R) d'insertion en trois dimensions pour insérer la partie d'extrémité distale (2C) vers une position cible (9G) à travers la lumière dans le corps du sujet (7), sur la base des données d'images en trois dimensions ;
**caractérisé en ce que** le dispositif médical (1, 1A, 1B, 1C, 1D, 1DA, 1E, 1F) comprend en outre un moyen (14) de génération d'image tomographique configuré pour générer une image tomographique (PA, PC, PS, PO) en deux dimensions sur la base de la position et de la direction de la partie d'extrémité distale (2C), à partir des données d'images en trois dimensions ; et
un moyen (12) de génération d'image superposée configuré pour générer une image superposée (PW1) sur laquelle l'image tomographique (PA, PC, PS, PO) en deux dimensions et la voie (R) d'insertion en trois dimensions sont affichées de manière superposée comme une image modèle en trois dimensions vue le long d'une ligne de visée (LA) arbitraire établie par un opérateur.

2. Dispositif médical selon la revendication 1, **caractérisé en ce que** le moyen (14) de génération d'image tomographique génère une image tomographique (PA, PC, PS, PO) sur un plan qui comprend une position de la partie d'extrémité distale (2C) et qui est perpendiculaire à une direction de la partie d'extrémité distale (2C).

3. Dispositif médical selon la revendication 1, **caractérisé en ce que** le moyen de génération d'image superposée superpose une marque (P2C) d'affichage de partie d'extrémité distale indiquant au moins l'une parmi la position et la direction de la partie d'extrémité distale (2C) sur l'image modèle en trois dimensions.

4. Dispositif médical selon la revendication 1, **caractérisé en ce que** le moyen de génération d'image superposée superpose sur la voie (R) d'insertion une marque (PJ1, PJ2, PJ3, PJ4) d'affichage de partie de bifurcation indiquant une position d'une partie de bifurcation de la lumière dérivée.

5. Dispositif médical selon la revendication 1, **caractérisé en ce que** le moyen de génération d'image superposée affiche la voie (R) d'insertion d'une manière telle qu'une partie de la voie (R) d'insertion allant d'une position de début d'insertion à la position de la partie d'extrémité distale (2C) est perceptible à partir d'une partie de la voie (R) d'insertion allant de la position de la partie d'extrémité distale (2C) à la position cible (9G).

6. Dispositif médical selon la revendication 1, **caractérisé en ce que** le moyen de génération d'image superposée n'affiche pas la partie de la voie (R) d'insertion allant de la position de début d'insertion à la position de la partie d'extrémité distale (2C).

7. Dispositif médical selon la revendication 1, **caractérisé en ce qu'**il comprend en outre :
un moyen (2B) de capture d'image configuré pour capturer une image endoscopique (RBS), le moyen (2B) de capture d'image étant prévu au niveau de la partie d'extrémité distale (2C) du moyen (2A) d'insertion ;
un moyen (13) de génération d'image endoscopique virtuelle configuré pour générer une image endoscopique virtuelle en utilisant une position, une direction, et un angle d'inclinaison de la partie d'extrémité distale (2C) en tant que paramètres de ligne de visée, à partir des données d'images en trois dimensions ; et
un moyen (4) d'affichage qui affiche au moins l'une parmi une image modèle en trois dimensions générée par le moyen de génération d'image superposée, l'image endoscopique, et l'image endoscopique virtuelle.

8. Dispositif médical selon la revendication 7, **caractérisé en ce que** le moyen (20, 20D) de calcul de position calcule la position et la direction de la partie d'extrémité distale (2C) sur la base des paramètres de ligne de visée de l'image endoscopique virtuelle qui est similaire à l'image endoscopique.

9. Dispositif médical selon la revendication 8, **caractérisé en ce que** le moyen de génération d'image superposée affiche une deuxième image (PR2) de voie d'insertion indiquant la voie d'insertion de manière superposée sur au moins l'une parmi l'image endoscopique et l'image endoscopique virtuelle.

10. Dispositif médical selon la revendication 9, **caractérisé en ce qu'**il comprend en outre :
un moyen (30) de calcul de zone d'affichage configuré pour calculer une zone d'affichage de la deuxième image (PR2) de voie d'insertion qui est affichée de manière superposée,
dans lequel lorsque la zone d'affichage est égale ou inférieure à une première valeur prédéterminée, le moyen de génération d'image superposée affiche la deuxième image (PR2) de voie d'insertion en surbrillance.

11. Dispositif médical selon la revendication 9, **caractérisé en ce qu'**il comprend en outre :
un moyen (30) de calcul de zone d'affichage configuré pour calculer une zone d'affichage de la deuxième image (PR2) de voie d'insertion qui est affichée de manière superposée ; et
un moyen (31) de calcul de voie d'insertion auxiliaire configuré pour calculer une voie (SR) d'insertion auxiliaire qui est une ligne transversale entre un plan incluant la voie d'insertion et une paroi luminale de la lumière,
dans lequel lorsque la zone d'affichage est égale ou inférieure à une deuxième valeur prédéterminée, le moyen de génération d'image superposée affiche une image (PSR) de voie auxiliaire indiquant la voie (SR) d'insertion auxiliaire de manière superposée.

12. Dispositif médical selon la revendication 9, **caractérisé en ce que** l'image modèle en trois dimensions générée par le moyen de génération d'image superposée est sélectionnée sur la base de la position de la partie d'extrémité distale (2C).

13. Dispositif médical selon la revendication 12, **caractérisé en ce que** le moyen (14) de génération d'image tomographique génère en outre une image tomographique (PA, PC, PS, PO) sur un plan qui comprend la position de la partie d'extrémité distale (2C) et qui est parallèle à la direction de la partie d'extrémité distale (2C).
